# EUROPEAN PATENT SPECIFICATION

(11) **EP 4 384 602 B1**
(45) Date of publication and mention of the grant of the patent: **25.03.2026**
(21) Application number: 22768261.4
(22) Date of filing: 10.08.2022
(51) Int. Cl.: C12N 5/078, A61K 35/19, C12N 15/113, A61P 35/00

(54) **PLATELETS TRANSFECTED WITH SIRNA AND THE THERAPEUTIC USES THEREOF**
MIT SIRNA TRANSFIZIERTE BLUTPLÄTTCHEN UND IHRE THERAPEUTISCHE VERWENDUNG
PLAQUETTES TRANSFECTÉES AVEC UN ARNSI ET LEURS UTILISATIONS THÉRAPEUTIQUES

(30) Priority: 11.08.2021 IT 202100021779
(43) Date of publication of application: 19.06.2024
(73) Proprietor: Plasfer S.r.l., 06126 Perugia (PG) (IT)
(72) Inventor: GRESELE, Paolo, 06132 Perugia (PG) (IT); MALVESTITI, Marco, 06034 Foligno (PG) (IT)
(74) Representative: Bianchetti & Minoja with Trevisan & Cuonzo IPS SRL
(86) International application number: PCT/EP2022/072484
(87) International publication number: WO 2023/017096

(56) References cited:
- WO-A1-2020/234868
- US-A1- 2018 177 727
- US-A1- 2020 248 185
- US-A1- 2020 323 964
- BUSCAIL LOUIS ET AL: "Role of oncogenic KRAS in the diagnosis, prognosis and treatment of pancreatic cancer", NATURE REVIEWS GASTROENTEROLOGY, NATURE PUBLISHING GROUP UK, LONDON, vol. 17, no. 3, 31 January 2020 (2020-01-31), pages 153 - 168, XP037041277, ISSN: 1759-5045, [retrieved on 20200131], DOI: 10.1038/S41575-019-0245-4

## Description

The present invention relates to platelets transfected with a siRNA designed to inhibit the mRNA of KRAS, a gene involved in carcinogenesis.

In particular, the invention provides a therapeutic composition comprising blood platelets transfected with siRNA targeting a mutant form of the KRAS oncoprotein. A further aspect of the invention relates to the use of the therapeutic composition for treatment of tumours in general, and pancreatic adenocarcinoma in particular.

### Background to the invention

The KRAS oncoprotein is a GTPase with the function of essential mediator of the intracellular signalling pathways involved in the growth and survival of tumour cells. In normal cells, KRAS acts as a molecular switch, alternating an inactive GDP-bound state with an active GTP-bound state. The transition between said states is facilitated by GTP hydrolysis catalysed by guanine nucleotide exchange factor (GEF), which loads GTP and activates KRAS, and by GTPase-activating protein (GAP), which inactivates KRAS. The bond between GTP and KRAS promotes the binding with the effectors which activate intracellular signalling pathways, including RAF-MEK-ERK (MAPK). Mutations of the KRAS gene are common in pancreatic cancer, pulmonary adenocarcinoma, colorectal cancer, gallbladder cancer, thyroid cancer and biliary cancer. KRAS mutations have been observed in about 94.1% of pancreatic tumours, and the KRASG12D mutation is the most frequent, with an incidence of 41%. It has been clearly demonstrated that mutations in the GTPase KRAS are commonly found in patients suffering from pancreatic ductal adenocarcinoma (PDAC), and said mutations are believed to be involved in the initial stage of oncogenesis, in tumour progression and in the spreading of metastasis. KRAS mutations in residues G12, G13 and Q61 are the most common mutations found in solid tumours. The somatic mutations activating KRAS are a distinguishing feature of some types of cancer, and interfere with the association with GAP, thereby stabilising the binding with the effectors and consequently increasing the KRAS signalling pathway. Patients suffering from tumours that present KRAS mutations have significantly worse outcomes and poor prognoses. For other types of tumour, inhibitors of various proteins of the MAPK intracellular signalling pathway (e.g. MEK, BRAF and EGFR) have been approved for clinical use, but so far, no molecules selective for mutated KRAS tumours exist for clinical use. Some treatments targeting the MAPK pathway have proved clinically ineffective in the treatment of tumours with the KRAS mutation. Moreover, a treatment not selectively targeting mutated forms of the specific tumour proteins can cause systemic toxicity due to inhibition of the signal induced by MAPK in normal cells.

There is consequently a need to develop treatments targeted selectively against the tumour which minimise or eliminate distribution of the drug in healthy cells.

The KRASG12D mutation is prevalent in pancreatic ductal adenocarcinoma (PDAC), a tumour characterised by a high mortality rate. PDAC patients with KRASG12D tumours have a particularly poor prognosis compared with those with other KRAS mutations. To improve the survival of patients with PDAC, it is therefore important to develop a novel therapeutic strategy targeting KRASG12D, which is more efficient and safer.

MicroRNAs, and their synthetic counterparts siRNAs, are small double-stranded non-coding RNAs which play an important role in post-transcriptional regulation of gene expression by suppressing the translation or induction of degradation of specific mRNAs.

The therapeutic use of siRNAs requires efficient methods for their delivery into the bloodstream, because they would otherwise be rapidly degraded and inactivated by plasmatic nucleases.

Methods that use bacteria, viruses, artificial synthetic vesicles (micelles, microsomes, etc.) and human cells (erythrocytes, macrophages, lymphocytes and stem cells) as carriers have been developed for this purpose.

Effective RNAi transport to the non-hepatic parenchymal organs, in particular the pancreas, remains a challenge. Although liposomes and nanoparticles can offer advantages for RNAi release compared with viral systems, they exhibit low efficiency and rapid elimination from circulation.

Approaches based on RNA interference (RNAi) targeting wild-type KRAS, or effectors downstream of the enzyme, which use nanoparticles as vectors, have exhibited a clear effect in the treatment of lung cancer, and in some colorectal cancer models.

However, targeting of KRAS for the treatment of pancreatic adenocarcinoma has so far been limited to administration of inhibitors by means of direct electroporation or biopolymer implants in pancreatic cancer xenotransplant models.

### State of the art

The use of human platelets to release siRNA is described in International application WO2014/118817, in the name of the present applicant. It reports some siRNA sequences targeting mutations of the KRAS oncogene, but not against the G12D mutation. In particular, the use of platelets loaded with siRNA against KRASG12D for the treatment of pancreatic adenocarcinoma is neither described nor suggested.

Patent publications WO2017/127473 and US2015/0307885 describe inhibition of expression of KRAS mutants by means of RNA interference, and dsRNA sequences useful for said purpose.

WO2010/001325 describes a polymer system suitable to delivery therapeutic agents, including siRNA.

### Description of the invention

It has now been found that blood platelets transfected with a siRNA specific for the KRAS G12D mutant, or microparticles deriving therefrom, effectively inhibit pancreatic cancer tumour growth *in vivo* in a murine model.

Platelets transfected with siRNA targeting KRASG12D prepared according to the present invention are able to reduce the tumour mass in animal models of a tumour expressing KRASG12D and they proved particularly effective in tests conducted in an animal model of pancreatic ductal adenocarcinoma (PDAC).

A first aspect of the invention therefore relates to a therapeutic composition comprising platelets transfected with a siRNA able to inhibit the mRNA of KRAS carrying the G12D mutation (KRASG12D), or platelet microparticles deriving therefrom, for use in the treatment of KRASG12D-expressing tumour, preferably pancreatic adenocarcinoma and more preferably pancreatic duttal adenocarcinoma
In a preferred embodiment, the sequence of the antisense strand of siRNA is complementary to an mRNA sequence of KRASG12D containing the mutated codon.

The siRNA molecule can contain 15 to 30, preferably 21, base pairs.

In a particularly preferred embodiment, the siRNA molecule comprises a sense strand and an antisense strand, wherein:
(i) the sense strand comprises or consists of the sequence 5'-GUUGGAGCUGAUGGCGUAGTT-3' (SEQ ID NO:1), and
(ii) the antisense strand comprises or consists of the sequence 5'-CUACGCCAUCAGCUCCAACTT-3' (SEQ ID NO:2).

The sequence of the platelet-transfected siRNA recognises the nucleotide substitution G→A in the sequence of the mutated KRAS gene (KRASG12D), and includes a 3' TT overhang to promote silencing efficiency. The central position of the mutated nucleotide increases the specificity of siRNA, thus preventing silencing of the wild-type KRAS gene and of genes carrying other types of mutation, such as KRAS G12C, KRAS G12N and KRAS G12V.

Transfection of platelets with siRNA is conducted according to the method described in EP2951292 (WO2014/118817), in the name of the same applicant. Briefly, platelets isolated from peripheral blood are placed in contact with siRNA in a medium containing ethyl alcohol and a polyamine selected from polyethylenimine and polylysine. To obtain the microparticles, after transfection the platelets are activated with suitable stimulating agents, for example by adding thrombin in the presence of calcium salts, as described in EP2951292.

The practice, used in transfusion medicine, of separating platelets and plasma from a donor enables the platelets to be harvested, transfected, resuspended in plasma and reinfused. Moreover, the short times required for platelet transfection allow intravenous reinfusion of the transfected platelets to the same individual from whom they were removed in a single session, thus eliminating the risk of alloimmunisation and rejection of the transfected platelets.

Thus in one preferred embodiment, the therapeutic treatment according to the present invention involves the collection of platelets from a cancer patient or a healthy donor, their transfection with siRNA and optional activation, and reintroduction into the patient of the transfected platelets and/or microparticles deriving therefrom.

The dose, route and frequency of administration of transfected platelets with siRNA KRASG12D able to reduce tumour growth have been determined in an animal model of PDAC.

In addition to the intravenous administration route used in the experimental tests *in vivo,* the intramuscular, intradermal or subcutaneous route, and/or administration *in situ,* can be employed for clinical use in humans.

In the *in vivo* tests, the frequency of administration was a total of 6 administrations in 14 days, but for clinical applications, one to six administrations can be performed.

The dose used in the animal model was about 45,000,000 platelets per administration. The human dose can be increased to 5x10¹¹ platelets; the dose range for clinical applications therefore preferably ranges between 45x10⁶ and 5x10¹¹ transfected platelets.

The invention is further illustrated in the examples below and in the annexed figures.

### Description of Figures

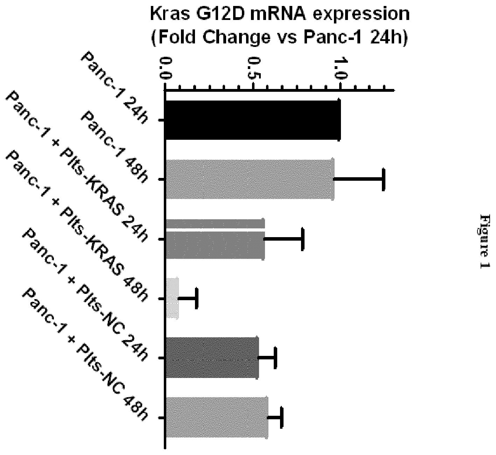

**Figure 1** *- Efficiency of inhibition of KRAS G12D by platelets transfected with siRNA targeting KRAS G12D in PANC-1 cells.*
   Platelets transfected with siRNA KRASG12D co-incubated *in vitro* with pancreatic adenocarcinoma cell line PANC-1 for 24 hours and 48 hours specifically reduce KRASG12D mRNA expression in the cell. The data are showed as levels of reduction of KRASG12D in the PANC-1 cells compared with the untreated PANC-1 cell; n = 3 independent experiments.
   Plts-NC: platelets transfected with siRNA Scrambled Negative Control DsiRNA (IDT, Inc.). Plts-KRAS: platelets transfected with siRNA KRASG12D.

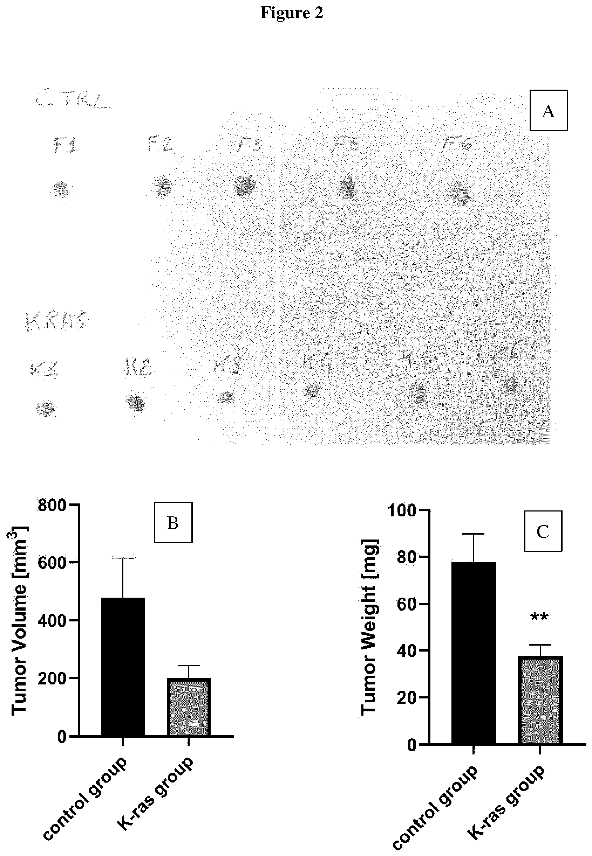

**Figure 2** - *Tumour mass in vivo - early treatment.*
   Analyses conducted after euthanasia of the animal; n = 6 mice per group. Control group: human platelets transfected with siRNA Scrambled Negative Control DsiRNA (IDT, Inc.). K-ras group: human platelets transfected with siRNA KRASG12D. A. Images of each explanted tumour. B. Tumour volume. C. Tumour weight.

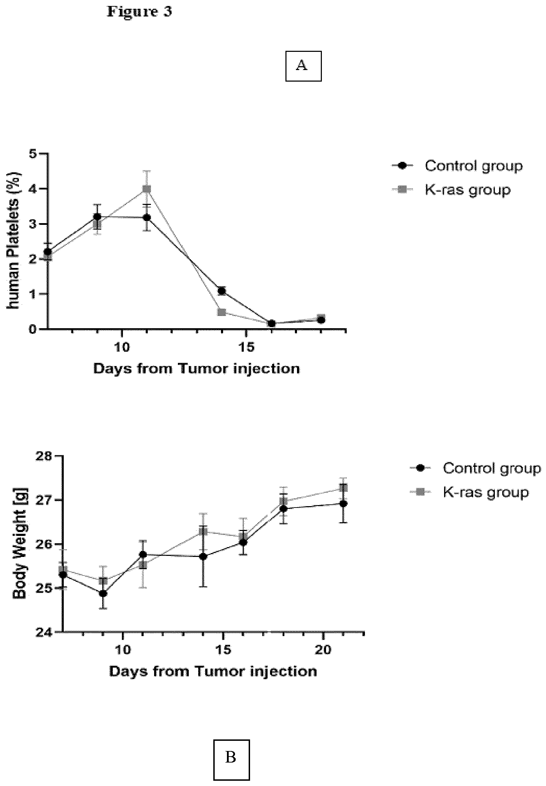

**Figure 3** *- Pharmacodynamics*
   A. Percentage of human platelets in total platelet population during treatment; n = 6 mice per group. B. Variation in percentage body weight of mouse during treatment (endpoint); n = 6 mice per group.

### Experimental part

### Materials and methods

### Preparation of human platelets with siRNA KRASG12D

Peripheral venous blood is drawn and collected into test tubes containing an anticoagulant.
1. The sample is centrifuged at 120 g for 10 minutes to obtain PRP (platelet-rich plasma).
2. The platelets are isolated from the plasma by common methods (platelet washing or gel filtration).
3. The platelets, thus isolated, are resuspended in RPMI 1640 with the addition of antibiotics (100 U of penicillin and 100 U of streptomycin), or in the donor's plasma, at a concentration ranging between 2x10⁵ and 1x10⁶ per microlitre, without exceeding the lower concentration limit of 130,000 platelets/microlitre, and 1 millilitre aliquots are transferred into the wells of a 24-well plate and placed under incubation at 37°C in a controlled atmosphere with 5% CO2.
4. The transfection medium is then prepared by inserting 32.4 microlitres of absolute ethyl alcohol, 32 microlitres of a polyamine selected from polylysine and polyethylenimine, and 168 microlitres of RPMI 1640 into a test tube, so as to reach the total amount of 200 microlitres in both cases.
5. After a 5-minute pause, siRNA KRASG12D at the concentration of 200 nM is added to said mixture, and after 15 minutes incubation at room temperature the solution is transferred to the well into which 1 ml of platelet suspension has already been introduced.
6. Transfection by centrifugation of the platelets (at 1000g for 10 minutes in the presence of PGI2 0.2 microM, and then resuspending them in 3 ml of RPMI 1640 culture medium) is interrupted after 5 minutes of incubation at 37°C.

### RealTime PCR

The cells were incubated with human platelets over different times, whereafter the RNA was reverse-transcribed with iScript Reverse Transcription Supermix for RT-qPCR (Bio-rad Laboratories) after total purification of the RNA with Trizol (Invitrogen), according to the manufacturer's instructions. The Quantitative PCR (qPCR) analyses were conducted with the AriaDX Real-time PCR System (Agilent) using SYBR Green Master Mix (Applied Biosystems). The relevant transcripts were standardised to RNA 18S transcription levels. Each reaction included three technical replicates, the average of which was calculated to define a biological replicate. The experiments were repeated three times on different days, and each experiment defined a biological replicate. Statistical analyses were conducted on ΔCt of biological replicates, and the results were expressed as the variation in relative increment. The primer sequences were as follows:
Human KRASG12D: forward 5' -ACTTGTGGTAGTTGGAGCAGA-3' (SEQ ID NO:3), reverse 5' -TTGGATCATATTCGTCCACAA-3' (SEQ ID NO:4).
18S: forward 5' -GCTTAATTTGACTCAACACGGGA-3' (SEQ ID NO:5), reverse 5' -AGCTATCAATCTGTCAATCCTGT-3' (SEQ ID NO:6).

### Animal model of pancreatic adenocarcinoma

Male NSG mice *Cg-Prkdc^{Scid}Il2rg^{tm1Wjl}*/SzJ (Charles Rivers) aged between 4 and 6 weeks were housed in ventilated cages with a cycle of 12 hours' light: 12 hours' darkness at 21-23°C and 40-60% humidity. The mice had free access to a diet and sterilised water. PANC-1 (10⁶ cells in 10 µl of PBS) were injected subcutaneously under general anaesthetic, using a 27 gauge syringe.

For the tumour mass analyses, Living Image version 4.4 (Caliper Life Sciences) was used to quantify the volume of all tumours. The exposure conditions (time, aperture, stage position and binning) were kept identical for all measurements within each experiment. The tumour mass was measured with an electronic balance (BL 224 Touch).

The mouse blood was isolated using heparin, with caudal samples. The blood (10 µl per mouse) was then diluted in 100 µl PBS. The samples were then incubated with Human CD41-FITC antibodies (BD Biosciences) and the corresponding controls, and analysed with a Cytoflex flow cytometer (Beckman Coulter). All the control samples were analysed together with the experimental samples.

### Results

The platelets transfected with siRNA targeting KRASG12D were co-incubated with PANC-1 cells for 24 and 48 hours, and the expression levels of KRASG12D mRNA in the human PANC-1 cells were evaluated with qPCR (Figure 1).

A first series of analyses was then conducted, relating to the *in vivo* efficacy data on a murine model of pancreatic cancer with mutated KRAS.

The platelets transfected with siRNA KRASG12D inhibited tumour growth *in vivo,* differently the platelets transfected with a siRNA designed not to recognise any target (siRNA NC) did not reduce the tumour growth..

NSG mice (NOD scid gamma mouse) were intravenously infused with human platelets starting from day 7 (start of early treatment) after subcutaneous injection of 1x10⁶ human PANC-1 cells (PDAC cell line) at day 0.

45x10⁶ human platelets were intravenously injected, and a total of 6 injections were administered to each mouse every 2-3 days from day 7 to day 21. The human platelets were isolated from 6 different healthy donors.

The tumour mass was quantified at the end of the study. The human platelet count in the mouse blood and the weight of the animal were constantly monitored during the experiment.

A reduction in the tumour mass was observed in the mice infused with platelets transfected with a siRNA against KRASG12D compared with those infused with platelets transfected with a siRNA NC, as demonstrated by the statistically significant reduction in tumour volume and mass illustrated in Figure 2.

Moreover, no significant variations were observed in the number of human platelets circulating in the blood of the mice (measured as percentage of human platelets/total number of platelets in the mouse blood), suggesting that the transfected platelets circulate efficiently in the host without any differences between the two experimental groups (Figure 3A).

Finally, no significant differences were observed between the two experimental groups as regards the variation in weight of the mice after treatment, confirming that the treatment is safe and does not cause undesirable side effects (Figure 3B).

## Claims

1. A therapeutic composition comprising platelets transfected with a siRNA able to inhibit expression of the mRNA coding for the KRAS protein carrying the G12D mutation (KRASG12D), for use in the treatment of a KRASG12D-expressing tumour.

2. The composition for use according to claim 1, wherein said KRASG12D-expressing tumour is pancreatic adenocarcinoma, preferably pancreatic ductal adenocarcinoma.

3. The composition for use according to claims 1-2, wherein said siRNA targets a KRASG12D mRNA sequence containing the mutated codon.

4. The composition for use according to claims 1-3, wherein said siRNA consists of 15 to 30 base pairs.

5. The composition for use according to claims 1-4, wherein said siRNA comprises a sense strand and an antisense strand, wherein:
(i) the sense strand comprises or consists of the sequence 5'-GUUGGAGCUGAUGGCGUAGTT-3' (SEQ ID NO:1), and
(ii) the antisense strand comprises or consists of the sequence 5'-CUACGCCAUCAGCUCCAACTT-3' (SEQ ID NO:2).

6. The composition for use according to claims 1-5, wherein said platelets are obtained by a transfection method which comprises contacting platelets isolated from peripheral blood with siRNA in a transfection medium containing ethyl alcohol and a polyamine selected from polyethylenimine and polylysine.

7. The composition according to claim 6, wherein said platelets are activated to produce microparticles.

8. The composition for use according to claims 1-7, wherein the tumour treatment comprises (i) harvesting platelets from the tumour patient or from a donor, (ii) transfecting the platelets with siRNA and (iii) (re)introducing the platelets into the patient.

## Patentansprüche

1. Therapeutische Zusammensetzung, umfassend Blutplättchen, die mit einer siRNA transfiziert sind, die in der Lage ist, die Expression der mRNA zu hemmen, die für das KRAS-Protein kodiert, das die G12D-Mutation (KRASG12D) trägt, zur Verwendung bei der Behandlung eines KRASG12D-exprimierenden Tumors.

2. Zusammensetzung zur Verwendung gemäß Anspruch 1, wobei der KRASG12D-exprimierende Tumor ein Adenokarzinom der Bauchspeicheldrüse, vorzugsweise ein duktales Adenokarzinom der Bauchspeicheldrüse ist.

3. Zusammensetzung zur Verwendung gemäß den Ansprüchen 1 bis 2, wobei die siRNA auf eine KRASG12D-mRNA-Sequenz gerichtet ist, die das mutierte Codon enthält.

4. Zusammensetzung zur Verwendung gemäß den Ansprüchen 1 bis 3, wobei die siRNA aus 15 bis 30 Basenpaaren besteht.

5. Zusammensetzung zur Verwendung gemäß den Ansprüchen 1 bis 4, wobei die siRNA einen Sense-Strang und einen Antisense-Strang umfasst, wobei:
(i) der Sense-Strang die Sequenz
5'-GUUGGAGCUGAUGGCGUAGTT-3' (SEQ ID NO: 1) umfasst oder aus ihr besteht, und
(ii) der Antisense-Strang die Sequenz
5'-CUACGCCAUCAGCUCCAACTT-3' (SEQ ID NO: 2) umfasst oder aus ihr besteht.

6. Zusammensetzung zur Verwendung gemäß den Ansprüchen 1 bis 5, wobei die Blutplättchen durch ein Transfektionsverfahren erhalten werden, das das Inkontaktbringen von aus peripherem Blut isolierten Blutplättchen mit siRNA in einem Transfektionsmedium umfasst, das Ethylalkohol und ein aus Polyethylenimin und Polylysin ausgewähltes Polyamin enthält.

7. Zusammensetzung nach Anspruch 6, wobei die Blutplättchen zur Herstellung von Mikropartikeln aktiviert werden.

8. Zusammensetzung zur Verwendung gemäß den Ansprüchen 1 bis 7, wobei die Tumorbehandlung (i) die Gewinnung von Blutplättchen von dem Tumorpatienten oder von einem Spender, (ii) die Transfektion der Blutplättchen mit siRNA und (iii) die (Wieder-) Einführung der Blutplättchen in den Patienten umfasst.

## Revendications

1. Composition thérapeutique comprenant des plaquettes transfectées avec un ARNsi capable d'inhiber l'expression de l'ARNm codant pour la protéine KRAS porteuse de la mutation G12D (KRASG12D), pour une utilisation dans le traitement d'une tumeur exprimant KRASG12D.

2. Composition pour une utilisation selon la revendication 1, dans laquelle ladite tumeur exprimant KRASG12D est un adénocarcinome pancréatique, de préférence un adénocarcinome du canal pancréatique.

3. Composition pour une utilisation selon les revendications 1 à 2, dans laquelle ledit ARNsi cible une séquence d'ARNm de KRASG12D contenant le codon muté.

4. Composition pour une utilisation selon les revendications 1 à 3, dans laquelle ledit ARNsi est constitué de 15 à 30 paires de bases.

5. Composition pour une utilisation selon les revendications 1 à 4, dans laquelle ledit ARNsi comprend un brin sens et un brin antisens, dans laquelle :
(i) le brin sens comprend ou est constitué de la séquence 5'-GUUGGAGCUGAUGGCGUAGTT-3' (SEQ ID NO : 1), et
(ii) le brin antisens comprend ou est constitué de la séquence 5'-CUACGCCAUCAGCUCCAACTT-3' (SEQ ID NO: 2).

6. Composition pour une utilisation selon les revendications 1 à 5, dans laquelle lesdites plaquettes sont obtenues par un procédé de transfection qui comprend la mise en contact de plaquettes isolées du sang périphérique avec un ARNsi dans un milieu de transfection contenant de l'alcool éthylique et une polyamine choisie parmi la polyéthylèneimine et la polylysine.

7. Composition selon la revendication 6, dans laquelle lesdites plaquettes sont activées pour produire des microparticules.

8. Composition pour une utilisation selon les revendications 1-7, dans laquelle le traitement tumoral comprend (i) la récolte de plaquettes du patient atteint de tumeur ou d'un donneur, (ii) la transfection des plaquettes avec un ARNsi et (iii) la (ré)introduction des plaquettes chez le patient.
